# EUROPEAN PATENT APPLICATION

(11) **EP 0 525 267 A2**
(43) Date of publication of application: **03.02.1993**
(21) Application number: 91402662.0
(22) Date of filing: 04.10.1991
(51) Int. Cl.: A61L 15/58, A61L 15/44

(54) **Prolonged occlusion treatment system**

(30) Priority: 02.08.1991 US 739429
(71) Applicant: FERNDALE LABORATORIES, INC., Ferndale, Michigan 48220 (US)
(72) Inventor: Shore, Ronald N., Potomac, Maryland 20854 (US); McMillan II, James T., Ferndale, Michigan 48236 (US)
(74) Representative: Phélip, Bruno

(57) **Abstract**

The healing of a number of skin disorders is effectively enhanced by prolonged continuous occlusion and/or hydration. Treating these disorders, e.g. psoriasis, is significantly facilited by occluding affected skin areas with a suitable barrier for a prolonged and continuous period of time. Such occlusion is effective by itself, but is materially improved by concurrent topical administration of medicament useful for treating the disorder. In such cases, the prolonged continuous occlusion markedly increases hydration which has the further therapeutic benefit of tremendously facilitating penetration of medicament. The provided barrier is one with low water-vapor transmission or one which is water impermeable. Within such framework, a treatment system has been devised which includes the application of medication, adhesive or adhesion enhancer and waterproof occlusive cover, and the removal of the occlusive cover, such as with an adhesive remover, after a prolonged period of at least three days. Special kits having key components have also been prepared for this treatment system.

## Description

### Related Applications

This application is directed to an improvement over the invention disclosed in USP 4,788,061, the entire text of which is incorporated herein by reference. This application is also a continuation-in-part of Application SN 281,665, filed October 28, 1988, which is a division of Application SN 752,406 (now USP 4,788,061), filed July 5, 1985.

### Field of the Invention

Occluding psoriatic lesions for an extended period of time without interruption, as described in the previously cited patent, can abate or relieve the symptoms of, alleviate the stress and suffering caused by and even heal such lesions completely. The noted beneficial effects are enhanced by concurrent application of medicament useful for treating psoriasis. The same approach is also useful for treating other skin disorders.

### Background

Emil G. Klarman confirms (Burger, A., "Medicinal Chemistry", second edition, page 1147, Interscience Publishers, Inc., New York, 1960) that there is no known cure for psoriasis, but "different therapeutic agents may produce involution of psoriatic lesions."

Prolonged, continuous occlusion (i.e., occlusion for at least three straight days without interruption), by itself or in conjunction with medication, has not been previously appreciated (by the medical community) as effective therapy for clearing psoriasis. Occlusion has been used on a daily or more frequent basis to enhance penetration of topical agents (e.g., Weinstein, Gerald D., et al., "A Clinical Screening Program for Topical Chemotherapeutic Drugs in Psoriasis", Arch. Dermatol., Vol. 117, pp. 388 to 393, July 1981; and Arndt, Kenneth A., "Manual of Dermatologic Therapeutics", third edition, pp. 143, Little, Brown and Company, 1983) and to prevent medication from staining clothing and bed sheets (e.g., Pearlman, Dale L., et al., "Paper-Tape Occlusion of Anthralin Paste", Arch. Dermatol., No. 5, pp. 625-630, May, 1984). However, the value of *prolonged, continuous, tight occlusion with a waterproof barrier* was neither recognized by these or other psoriasis experts nor employed therapeutically by physicians for healing and clearing psoriasis. Although occlusion of psoriatic lesions with an impermeable barrier can cause reformation of the granular cell layer (Fry, L., Almeyda, J., McMinn, R.M.H., "Effect of Plastic Occlusive Dressings on Psoriatic Epidermis", Br., J. Dermatol., 82, 458 to 462, 1970), *daily* reapplication of an occlusive cover was not found to be an effective treatment. The authors stated in their concluding paragraph, "It is not suggested that occlusion by itself should ever be used to treat psoriasis...". Furthermore, in the past, some patients with psoriasis may have avoided using adhesive tape on their skin since it was known "that its removal may traumatize the epidermis sufficiently to evoke an isomorphic lesion" (Van Scott, Eugene J., and Farber, Eugene M., "Dermatology in General Medicine", Chapter 8, page 226, McGraw-Hill Book Company, 1971).

Weinstein et al., applied a large series of test agents to psoriatic plaques under occlusion daily for up to nine days. These investigators, who included a distinguished group of psoriasis experts from multiple university centers, were searching for new drug treatments for psoriasis. They used repeated *daily* occlusion to enhance penetration of drugs they were evaluating. They did not report any significant benefit of daily occlusion itself in clearing psoriasis.

Williamson found that clearing of resistant plaques with anthralin was significantly enhanced by using an occlusive dressing (occluded for 12 hours daily with PVC film), but irritant side effects were increased (Coskey, Ralph, J., "Dermatologic Therapy", Dermatology, Vol. 11, No. 1, pages 25 to 52, at 37, July, 1984). Pearlman, et al., devised a practical method for outpatient therapy of psoriasis by instructing patients to cover nightly anthralin paste applications with semipermeable paper tape. The tape prevented anthralin staining of pajamas and bed sheets but did *not* improve the efficacy of the treatment.

A medicated adhesive tape for treating various steroid-responsive dermatoses, including psoriasis, is being marketed. The "Physicians' Desk Reference" (PDR), 39th edition, page 897, 1985, provides the following information:
Cordran® Tape is a transparent, inconspicuous, plastic surgical tape, impervious to moisture. It contains... (flurandrenolide, Dista), a potent corticosteroid for topical use... .
Each square centimeter contains 4 mg. of flurandrenolide uniformly distributed in the adhesive layer. The tape is made of a thin, matte-finish polyethylene film which is slightly elastic and highly flexible.
The adhesive is a synthetic copolymer of acrylate ester and acrylic acid which is free from substances of plant origin. The pressure-sensitive adhesive surface is covered with a protective paper liner to permit handling and trimming before application... .
The tape serves as both a vehicle and an occlusive dressing. Retention of insensible perspiration by the tape results in hydration of the stratum corneum and improved diffusion of the medication. The skin is protected from scratching, rubbing, desiccation, and chemical irritation. The tape acts as a mechanical splint to fissured skin. Since it prevents removal of the medication by washing or the rubbing action of clothing, the tape formulation provides a sustained action... ."
The directions for use state:
Replacement of the tape every twelve hours produces the lowest incidence of adverse reactions; but it may be left in place for twenty-four hours if it is well tolerated and adheres satisfactorily.

Arndt's text states on page 143 that overnight or 24-hour occlusive therapy with Cordran® Tape will initiate involution in most lesions, and corticosteroids exert their beneficial effects in this setting as mitotic inhibitors. The text further asserts on page 274 that occlusive (airtight) dressing increases efficacy of cream preparations in treating psoriasis. However, there is no mention of prolonged occlusion treatment to treat psoriasis, and on page 274 the text points out potential undesirable effects from occlusive therapy.

An advertisement for Diprolene® ointment for treating psoriasis has a specific instruction not to use an occlusive dressing (Journal of the American Academy of Dermatology, Vol. 12, No. 4, 101A, April, 1985). It is well known that extremely potent corticosteroids, such as Diprolene, may cause skin atrophy and other side effects, particularly if used with daily occlusion.

A dermatologic patch used in conjunction with a topical steroid cream produced a significant degree of lesion resolution in patients with chronic plaque-type psoriasis. However, sites treated with the same patch alone, *changed every forty-eight hours* for three weeks, showed insignificant change (Wilkinson, R.D., Ohayon, M., "Therapeutic Response to a Dermatologic Patch and Betamethasone Valerate 0.1 Percent Cream in the Management of chronic Plaques in Psoriasis", Cutis, Vol. 45, No. 6, pages 468 to 470, June, 1990). The authors state, "The failure of hydrocolloid patch alone to reverse psoriasis was unexpected. Shore (Shore, R.N., Clearing of psoriatic lesions after the application of tape. N. Eng. J. Med. 312, 246-247, 1985) noted that tape occlusion usually caused regression of psoriatic lesions." The authors, who were aware of Shore's research, failed to appreciate the importance of employing prolonged continuous occlusion (i.e., occlusion for at least three straight days without interruption) and did not achieve the significant results reported by Shore.

Applicant has been assured by an expert in the adhesive tape art that, for at least 20 years, the technology has been known for producing:
a) adhesive tape which has low water-vapor (moisture-vapor) transmission or is waterproof,
b) adhesive tape which is flexible,
c) adhesive tape which is highly elastic, and
d) adhesive tape which has a very aggressive adhesive which is routinely capable of securing the tape tightly to skin for at least seven days, despite body movement.

Applicant has also been assured that, prior to July 5, 1985, anyone of ordinary skill in the adhesive tape art could produce, with ease, adhesive tape with any combination of properties (a) through (d) if the desire or incentive to do so existed.

Incorporating in a surface or in adhesive on a surface of an occlusive cover (a) an agent which suppresses bacterial growth and/or b) a topical medicament for treating psoriasis and/or c) means to protect skin from irritation by adhesive, adhesion enhancer or prolonged occlusion is readily within the skill of the art. No problem exists in combining such agents in said adhesive surface as long as selected agents are compatible with each other.

### Summary of the Invention

In direct conflict with standard accepted approach of daily (or more frequent) dressing changes in the topical treatment of dermatologic diseases, this invention requires occluding affected skin areas by continuously maintaining a tight barrier thereover for a period of at least three days. The tight barrier is, e.g., in the form of a waterproof occlusive cover which preferably has adhesive and optionally has suitable topical medication on its skin-facing surface. An object of this invention is to abate or relieve the symptoms of, alleviate distress and suffering caused by and to heal skin disorders, e.g. psoriatic lesions.

Throughout the disclosure, all references to "tape" or to "adhesive tape" actually cover all corresponding waterproof occlusive dressings, whether or not they are technically regarded as "tapes".

Although this invention is primarily concerned with a waterproof occlusive cover or dressing useful for treatment of psoriasis, it is not so limited. The prolonged occlusive therapy is also effective for other difficult-to-treat skin problems, such as keloids (i.e., thick raised scars), fissured hand dermatitis, granuloma annulare, lichen planus, calluses, parapsoriasis and other thickened-skin disorders (well known to practicing dermatologists) for which occlusion enhances medicament, e.g. steroid, penetration and/or hydration positively aids the healing process. When a corticosteroid is concurrently applied topically, prolonged occlusion enhances penetration of the drug in all such cases and provides significant hydration. This therapy is far more effective than, e.g., plastic gloves, which do not provide continuous tight occlusion on all injured parts of the hands.

One aspect of this invention concerns a treatment system, referred to as a "Prolonged Occlusion Treatment System", which employs a series of steps and several different materials to maximize the effectiveness of prolonged occlusion for the treatment of psoriasis and other skin disorders.

Another aspect of this invention comprises a kit which is made up of components particularly suited for achieving the treatment system. The kit is also referred to as a "Prolonged Occlusion Treatment System".

A further aspect of the invention involves particular items of the kit, which includes an adhesive or adhesive enhancer and a remover thereof.

Further objects of the invention will be apparent from the following details.

### Details

The mere continuous maintenance of, e.g., waterproof adhesive tape, in tight contact with and over a skin surface afflicted with psoriasis (or other skin disorder for which hydration positively aids the healing process) for a period of at least three days results in noticeable improvement, including clearing of the skin and alleviation of the stress and suffering caused by thus-occluded lesions. When an adhesive tape is employed, the tape is, optionally, of any suitable material, such as plastic film. It is essential to have a waterproof occlusive cover or at least one with low moisture vapor transmission which will result in substantial skin hydration. A tape which is not waterproof, such as paper tape, is totally ineffective. The tape should be flexible so that it will conform to the surface to which it is applied and, preferably, accommodate movement of limbs to which it is secured; it should be able to stretch and is advantageously elastomeric.

Although adhesive on currently-available waterproof adhesive tape is not designed to maintain the tape secured to human skin routinely for a period of at least seven days despite body movement, available products can assist marketed adhesive tapes to achieve that goal. Such products are adhesives or adhesion enhancers which must not irritate the surface to which they are applied. They must be inert to or, at least, not adversely reactive with medicament applied to affected skin areas. Whether or not any other medicament is used on or in conjunction with the occlusive dressing, the occlusive dressing advantageously contains (on the skin-facing surface) a component, such as zinc oxide, which suppresses overgrowth of skin bacteria and associated odor due to marked hydrating effects of tape applied continuously for a prolonged period (a week or longer). The adhesion enhancer is, optionally, a skin protectant, particularly when the occlusive dressing is a waterproof adhesive tape, the adhesive of which is adequate to maintain the tape routinely on skin for at least one week irrespective of body movement. The skin protectant is designed to reduce irritation otherwise attributable either to the adhesive and/or to the tight occlusion for a prolonged period.

The adhesives or adhesion-enhancers are actually adhesives, protectants and/or degreasers designed to be applied to skin prior to application of tapes, bandages, dressings and skin closure strips, e.g., Steri-Strip®. Suitable embodiments include adhesive products, such as Mastisol® and Dermastick®; skin protectants, such as tincture of benzoin, compound tincture of benzoin, and Skin Prep®; and degreasers, such as Freon®. Removers for the adhesive or adhesion enhancer include aliphatic and aromatic hydrocarbon, alcohol, ketone and aldehyde based adhesive removers and solvents.

Throughout this disclosure reference is made specifically to psoriasis and to psoriatic lesions. All such references are equally applicable to other skin disorders for which occlusion enhances medicament penetration and/or hydration positively aids the healing process. When occlusive therapy is combined with topical medicament administration, the hydration further enhances drug penetration and effectiveness.

The invention is not merely an occlusive cover, separately-applied adhesive or adhesion enhancer and/or a specific medicament that is optionally applied under the occlusive cover and/or incorporated in its skin-contacting surface. The invention lies in means for tightly occluding psoriatic lesions (or skin affected by some other disorder for which hydration positively aids the healing process and/or for which occlusion enhances medicament penetration) for a continuous period of at least three days, preferably at least seven days and extending to, e.g., three weeks. (The same procedure, however, is relatively ineffective when the occlusive cover is changed every forty-eight hours or more frequently, as was noted by both Wilkinson, et al., and Fry, et al.)

The procedure is effected by covering, e.g., psoriatic lesions on a skin surface with a waterproof occlusive cover, such as tape, and maintaining the occlusive cover in place for the noted period of time. A separate adhesive or adhesion enhancer is applied around an affected skin area prior to the application of the occlusive cover. The adhesive or adhesion enhancer must be one which is capable of maintaining the occlusive cover in place and in tight occlusion for the desired duration. It should preferably function as well to reduce or prevent irritation from the occlusive cover. The process is enhanced by providing the skin-contacting side of the occlusive cover with medicament useful for treating psoriasis. The medicament is optionally coated on the skin-contacting side of the occlusive cover in an appropriate amount. Alternatively, such medicament is applied, e.g., directly to the psoriatic lesions prior to applying the occlusive cover, such as tape, thereover.

The separate adhesive or adhesion enhancer must be one which, in combination with adhesive (if any) on the tape, is adequate to maintain the tape routinely in tight occlusion on skin for at least one week and preferably for at least three weeks.

When medicament is employed in combination with occlusion, such medicament is preferably in ointment form, rather than in cream, lotion, gel or other form, but can be in any form that is not irritating under an occlusive cover. Before an occlusive cover and medicament are reapplied, involved areas should be washed well. Soaking for thirty minutes or more, immediately before retreatment, is particularly helpful on more resistant sites. Fissures and pustular psoriasis lesions tend to respond particularly well to this therapy. Total clearing is common, but, even if such lesions are not totally eradicated, there is usually such improvement and relief of discomfort that patients are enormously grateful.

Prolonged occlusion therapy has advantages over other topical psoriasis treatments. Total clearing and remission is possible and frequent, which is certainly not true with topical corticosteroids alone, currently the most popular out-patient treatment for psoriasis. There is no staining, no mess and no odor from medications. Because medication is applied infrequently, it is very economical.

Those patients with minimal (less than 10 to 15 percent of the body surface) skin involvement, i.e., the great majority of patients with psoriasis, would benefit most from this therapy. Patients with relatively small numbers of plaques (mainly on the trunk and extremities) are particularly good candidates.

The particular treatment system of this invention comprises four steps:
1) A thin coating of medication, such as corticosteroid ointment, is applied to lesion(s) selected for treatment.
2) An adhesive or adhesion enhancer, such as Mastisol® or tincture of benzoin, is applied around the selected lesion(s). This will serve to enhance adhesion of tape (step 3) and to prevent irritation from the tape and its adhesive.
3) A waterproof tape in strip, patch or other form is applied singly or in groups over the selected lesions and the surrounding adhesive or adhesion enhancer.
4) After a prolonged period, i.e., at least three days, but preferably a week or more, the tape and all remaining adhesive and skin residue is removed from the skin, using an adhesive remover, such as Detachol®
If the treated lesion has not totally resolved with this treatment, steps (1) through (4) are repeated as needed until full resolution is achieved.

The key materials necessary for this treatment regimen, namely: the adhesive or adhesion enhancer, the occlusive cover and the adhesive remover, are, optionally, packaged in a kit or as individual components. The adhesive or adhesion enhancer must be one, which is pharmacologically-acceptable when topically administered to human skin. It must also have sufficient bonding or bond-enhancing strength to maintain an ordinary tape dressing routinely in place, under normal conditions, on skin for a period of at least three days and preferably for a period of up to one or even three weeks, even when securing tape to portions of the body which experience continual and/or repeated stress, such as elbows. The adhesive or adhesion enhancer must also be fairly easily removable from the skin without injuring skin tissue. Likewise, the adhesive remover must be one which is pharmacologically-acceptable for topical application to the skin.

A kit of this invention comprises the adhesive or adhesion enhancer, occlusive cover, which is preferably flexible and elastic, and the adhesive remover. The kit optionally further contains medicament, either as a separate component or incorporated into the occlusive cover, for treating lesions to which the occlusive treatment system is applied.

The invention and its advantages are readily understood from the preceding description. Various changes may be made in the specific adhesive or adhesion enhancer, the selected occlusive cover, the particular adhesive remover and/or the medicament without departing from the spirit and scope of the invention or sacrificing its material advantages. The materials hereinbefore described and the specified method are merely illustrative of preferred embodiments of the invention.

## Claims

1. A process for treating a skin disorder for which healing or improvement is significantly aided by prolonged, continuous occlusion and/or hydration which comprises: (a) applying a skin-compatible adhesive or adhesion enhancer around a lesion of the skin disorder, such adhesive or adhesion enhancer being one which prevents irritation from an occlusive cover or dressing applied thereover, (b) applying waterproof occlusive cover or dressing over the lesion and surrounding adhesive or adhesion enhancer, and (c) manually removing the occlusive cover or dressing after a prolonged period of at least three days.

2. A process according to claim 1 which further comprises removing all remaining adhesive or adhesion enhancer after removing the occlusive dressing.

3. A process according to claim 2 wherein the occlusive cover or dressing has incorporated therein skin protectant means to reduce irritation.

4. A process according to claim 1 wherein the occlusive cover or dressing has a skin-contacting side comprising adhesive means for routinely securing said occlusive cover or dressing tightly to skin for at least seven days despite body movement.

5. A process according to claim 1 wherein the adhesive or adhesion enhancer is capable of routinely securing the occlusive cover or dressing tightly to skin for at least seven days despite body movement.

6. A process according to one of claims 1 to 5 which further comprises, prior to step (b), applying to the lesion of the skin disorder a thin coating of medication for treating said disorder.

7. A process according to claim 6 wherein the prolonged period is at least one week.

8. A process according to claim 6 wherein the occlusive dressing and the adhesive are removed with an adhesive remover.

9. A process according to claim 6 wherein the thin coating of medication is on a surface of the waterproof occlusive dressing.

10. A process according to claim 6 wherein the waterproof occlusive dressing has a lesion-facing surface comprising an agent which suppresses bacterial growth.

11. A kit useful for a prolonged occlusion treatment system and which comprises a waterproof occlusive cover or dressing, a topically-acceptable adhesive or adhesion enhancer routinely capable of maintaining the occlusive cover or dressing in tight occlusion on skin for at least three days, and topically-acceptable remover means for removing the adhesive or adhesive enhancer from skin.

12. A kit according to claim 11 which comprises topically-acceptable adhesive.

13. A kit according to claim 11 which comprises topically-acceptable adhesion enhancer.

14. A kit according to claim 11 wherein the adhesive or adhesion enhancer is routinely capable of maintaining the occlusive cover or dressing in tight occlusion on skin for at least one week.

15. A kit according to claim 11 wherein the adhesive or adhesion enhancer is Mastisol®.

16. A kit according to claim 11 which further comprises medicament useful for treating a skin disorder for which healing or improvement is significantly aided by prolonged, continuous occlusion and/or hydration.

17. A kit according to claim 11 wherein the waterproof occlusive cover or dressing has a surface comprising medication for treating a skin disorder for which healing or improvement is significantly aided by occlusion and/or hydration.

18. A kit according to claim 11 wherein the waterproof occlusive cover or dressing has a skin-contacting side comprising adhesive means for routinely securing said occlusive cover or dressing tightly to skin for at least seven days despite body movement.

19. A kit according to claim 11 wherein the waterproof occlusive cover or dressing has a surface comprising an agent which suppresses bacterial growth.

20. A kit according to claim 11 wherein the waterproof occlusive cover or dressing is flexible and elastic.

21. A kit according to claim 11 wherein the waterproof occlusive cover or dressing is elastomeric.

22. A kit according to claim 11 wherein the adhesive remover is Detachol®.
